(19) Europäisches Patentamt / European Patent Office / Office européen des brevets

(11) **EP 4 772 661 A1**

(12) **EUROPEAN PATENT APPLICATION**
published in accordance with Art. 153(4) EPC

(43) Date of publication:
**08.07.2026 Bulletin 2026/28**

(51) International Patent Classification (IPC):
**C22C 18/00** (2006.01)　　**C22C 18/02** (2006.01)
**C22C 18/04** (2006.01)　　**C22C 1/00** (2023.01)
**C22F 1/16** (2006.01)　　**C22F 3/02** (2006.01)
**C22F 1/00** (2006.01)　　**A61L 27/04** (2006.01)
**A61L 27/50** (2006.01)

(21) Application number: **24935720.3**

(22) Date of filing: **10.12.2024**

(86) International application number:
**PCT/CN2024/137960**

(87) International publication number:
**WO 2025/218192 (23.10.2025 Gazette 2025/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **17.04.2024 CN 202410463951**

(71) Applicant: **University Of Science And Technology Beijing**
**Beijing 100083 (CN)**

(72) Inventors:
- WANG, Luning
  **Beijing 100083 (CN)**
- SHI, Zhangzhi
  **Beijing 100083 (CN)**
- LI, Meng
  **Beijing 100083 (CN)**
- ZHOU, Chao
  **Beijing 100083 (CN)**
- ZHAO, Shanpeng
  **Beijing 100083 (CN)**

(74) Representative: **JD&P Patent Attorneys**
**Joanna Dargiewicz & Partners**
**ul. Mysliborska 93A/50**
**03-185 Warszawa (PL)**

(54) **HIGH-PERFORMANCE NEAR-PURE ZINC ALLOY, PREPARATION METHOD THEREFOR AND USE THEREOF**

(57) A high-performance near-pure zinc alloy, and a preparation method and use thereof are provided, belonging to the technical field of zinc alloys. The high-performance near-pure zinc alloy includes a Zn content not less than 99.5%, with a balance being at least two trace alloying elements selected from the following 14 elements: Mg, Mn, Li, Cu, Ag, Ca, Sr, Fe, Zr, Ti, Al, C, Ga, and Si. The high-performance near-pure zinc alloy has a yield strength (YS) not less than 250 MPa, an ultimate tensile strength (UTS) not less than 320 MPa, an elongation to failure (EL) not less than 25%, a variation in mechanical properties after one year of room-temperature storage not greater than 5%, a maximum bending force not less than 2 kN, and a stress corrosion susceptibility factor not greater than 15%. The high-performance near-pure zinc alloy exhibits a size of corrosion pits not greater than 15 $\mu$m after 60 d of immersion, with a standard deviation not greater than 3 $\mu$m. The high-performance near-pure zinc alloy can be can be employed in the manufacture of biodegradable stents, bone-implant devices, intestinal/vascular/neural anastomotic staplers or staples, vascular clips, guided bone-regeneration (GBR) membranes, guided tissue-regeneration (GTR) membranes, artificial heart valves, dura-mater repair membranes, dissolvable bridge plugs and dissolvable fracturing balls for petroleum drilling, zinc-alloy die castings, galvanized zinc materials, architectural zinc materials, battery-grade zinc materials, printing-grade zinc materials, and zinc-based brazing alloys.

EP 4 772 661 A1

**FIG. 1**

**Description**

**TECHNICAL FIELD**

The present disclosure belongs to the technical field of zinc alloys, and relates to a high-performance near-pure zinc alloy, and a preparation method and use thereof.

**BACKGROUND**

Zinc, an essential trace element in the human body, promotes osteoblast proliferation and differentiation while exhibiting anti-inflammatory and antithrombotic properties. An optimal degradation rate of Zn meets the requirements for orthopedic and coronary implant devices, gradually degrading post-functionality to eliminate long-term risks associated with secondary surgical removal or permanent retention of implants, common drawbacks of traditional materials. Consequently, zinc stands as a potential next-generation biodegradable metallic material for medical applications.

Driven by the trend toward lightweight and highly biocompatible medical devices (e.g., bone nails, coronary stents), demands for enhanced material strength, plasticity, and stress corrosion resistance have intensified. The mechanical properties of pure zinc remain inadequate, showing strength less than 100 MPa, which is far inferior to the requirements for biodegradable metallic implants. Alloying represents an effective strategy to strengthen pure zinc, yet current approaches rely on increasing alloying element content. For instance, Tong et al. have reported a Zn-4.5Ge alloy with 4.5 at.% total alloying elements, achieving an ultimate tensile strength (UTS) of 237 MPa (X Tong, et al. Acta Biomaterialia, 2018, 82: 197-204). Similarly, Mostaed et al. have developed a Zn-2.5Ag-0.6Mn alloy (3.1 at.% total alloying elements) with UTS of 302 MPa and elongation to failure (EL) of 35% (E Mostaed, et al. Acta Biomaterialia, 2020, 105: 319-335). However, continuous increases in alloying content yield diminishing performance returns while escalating resource dependency and material costs. Tang et al. have demonstrated this trade-off: a Zn-3.1 at.%Cu alloy exhibits UTS of 257 MPa and EL of 47%, but adding 2.6 at.%Mg increases the UTS to 440 MPa at the cost of plummeting EL to 1%, raising total alloying elements to 5.7 at.% (Z B Tang, et al. Materials and Design, 2017, 117: 84-94).

While boosting alloying element content to enhance strength aligns with conventional design principles, highly alloyed zinc-based implants risk triggering critical issues.

(1) Excessive release of alloying elements into surrounding tissues may cause adverse effects if not metabolized promptly.

(2) Intermetallic second phases formed between alloying elements and Zn increase in volume fraction with higher alloying content. These phases degrade slower than the Zn matrix, leading to prolonged residual degradation products *in vivo*. Simultaneously, micro-galvanic couples between second phases and the Zn matrix accelerate localized corrosion. Accumulated corrosion products attract inflammatory cells and macrophages, hindering rapid clearance and tissue homeostasis, thereby degrading biocompatibility.

(3) Abundant second phases in highly alloyed Zn alloys promote localized corrosion. Under stress corrosion conditions, stress concentrations at phase-matrix interfaces initiate cracks, exacerbating localized corrosion and causing premature device failure. Bowen et al. have observed acute local inflammation (neutrophil/eosinophil infiltration) in rat aortas three months after implanting Zn-$x$Al alloys ($x$=2.4, 7, 11.3 at.%), with inflammation severity scaling with Al content. Zn-Al alloys exhibit intergranular corrosion in aortic service, risking implant fracture and failure. Furthermore, delayed metabolism of high-dose Al may induce neurotoxicity (P K. Bowen, et al. Journal of Biomedical Materials Research, 2018, 106: 245-258).

Thus, overcoming existing design limitations to develop medical-grade biodegradable zinc alloys that deliver high strength and plasticity, stress corrosion resistance, superior biocompatibility, and uniform degradation, while minimizing alloying elements, remains a critical unmet challenge.

**SUMMARY**

The Chinese National Standard GB/T 470-2008 "Zinc Ingots" classifies zinc ingots into five grades based on chemical composition: Zn99.995, Zn99.99, Zn99.95, Zn99.5, and Zn98.5, with purities of 99.995%, 99.99%, 99.95%, 99.5%, and 98.5%, respectively. Addressing the limitations of existing zinc alloys such as high alloying element content (abundant intermetallic second-phase particles), sluggish performance enhancement, susceptibility to stress corrosion cracking, and poor biocompatibility, the present disclosure provides a high-performance near-pure zinc alloy, and a preparation method and use thereof. The near-pure zinc alloy maintains high zinc purity (volume fraction of second-phase particles not greater than 2%) while delivering superior performance. The term "near-pure zinc alloy" embodies three defining characteristics:

(1) In atomic percent (at.%), the near-pure zinc alloy includes not less than 99.5% Zn, with a balance including trace alloying elements intentionally added.

(2) The near-pure zinc alloy exhibits a yield strength (YS) not less than 250 MPa, an ultimate tensile strength (UTS) not less than 320 MPa, an elongation to failure (EL) not less than 25%, a variation in mechanical properties after 1 year of room-temperature storage not greater than 5%, a maximum bending force not less than 2 kN, and a stress corrosion susceptibility factor not greater than 15%.

(3) Regarding degradation behavior, the near-pure zinc alloy exhibits high degradation uniformity, showing an average size (in equivalent diameter) of corrosion pits not greater than 15 $\mu$m after 60 days (the unit will hereafter be abbreviated as "d") of immersion, with a standard deviation not greater than 3 $\mu$m and a uniformity coefficient ($\eta$) of pitting distribution not less than 80%. The uniformity coefficient $\eta$ is calculated as follows:

$$\eta = 100 \times (1 - \frac{\sum_{i=1}^{N} |p_i - \bar{p}|}{N\bar{p}}) \tag{1}$$

$p_i$ denotes the number of corrosion pits in the i-th grid, $\bar{p}$ represents the average number of corrosion pits, and *N* is the total number of grids.

The present disclosure is implemented through the following technical solutions.

A first aspect of the present disclosure is to provide a high-performance "near-pure zinc alloy", where

The high-performance near-pure zinc alloy includes a Zn content not less than 99.5%, with a balance being at least two trace alloying elements selected from the following 14 elements: Mg, Mn, Li, Cu, Ag, Ca, Sr, Fe, Zr, Ti, Al, C, Ga, and Si.

The second-phase particles in the near-pure zinc alloy have a volume fraction less than or equal to 2% and a size (in equivalent diameter) less than or equal to 1 $\mu$m.

By precisely controlling the addition of alloying elements and employing a combined severe plastic deformation and rapid cooling process, a co-segregation structure including two or more elements selected from the aforementioned 14 elements is established at defect sites such as grain boundaries, subgrain boundaries, twin boundaries, and dislocations, and does not form second phases at these defect sites.

The total content of alloying elements in this near-pure zinc alloy is not greater than 0.5% (in atomic percent), effectively mitigating the issues prevalent in highly alloyed zinc systems, such as delayed metabolism of alloying elements, long-term retention of second-phase particles, and compromised biocompatibility.

The co-segregation structure formed by multiple trace alloying elements in the near-pure zinc alloy delivers four critical functions:

(1) Effectively impeding dislocation motion and interface (e.g., grain boundary) migration, significantly enhancing the near-pure zinc alloy's strength while maintaining excellent mechanical stability;

(2) Strengthening interfaces to improve stress corrosion resistance and suppress intergranular cracking;

(3) Stabilizing interfaces to transition the alloy's plastic deformation mode from dislocation slip-dominated to grain boundary sliding-dominated, dramatically improving plasticity; and

(4) Attenuating micro-galvanic corrosion effects, thereby significantly enhancing degradation uniformity.

The microstructure of the "near-pure zinc alloy" exhibits two defining characteristics:

(1) Zn grain size averaging not greater than 2 $\mu$m, second-phase particle volume fraction not greater than 2%, second-phase particle size (in equivalent diameter) not greater than 1 $\mu$m, and dislocation density not greater than $2 \times 10^{14}$ m$^{-2}$.

(2) After holding in air atmosphere at 37°C to 200°C for 5 h to 100 h, the average size of Zn grains is not greater than 10 $\mu$m, demonstrating exceptional microstructural stability.

The recrystallization temperature of this "near-pure zinc alloy" measures not less than 80°C, significantly exceeding that of pure Zn (15°C).

Room-temperature tensile properties of the "near-pure zinc alloy" include: YS of 250 MPa to 450 MPa, UTS of 320 MPa to 650 MPa, and EL of 25% to 185%.

After one year of storage in 37°C air, the "near-pure zinc alloy" exhibits strength reduction not greater than 5% and elongation reduction not greater than 5%.

Maximum bending force of the "near-pure zinc alloy" reaches not less than 2 kN.

Room-temperature compressive properties of the "near-pure zinc alloy" demonstrate: YS of 260 MPa to 510 MPa, stress at 70% compressive deformation 750 MPa to 980 MPa (compressive strength not less than 750 MPa).

Degradation of the "near-pure zinc alloy" in simulated body fluid (SBF), Hanks' solution, phosphate-buffered saline (PBS), and saline solution at 37°C proceeds uniformly. Post 60-d immersion, corrosion pit size (in equivalent diameter) is not greater than 15 $\mu$m in average with standard deviation not greater than 3 $\mu$m. Grid-count analysis confirms that the uniformity coefficient ($\eta$) of pitting distribution is not less than 80%, with degradation rates of (0.02-1.80) mm/year.

Stress corrosion susceptibility factor of the "near-pure zinc alloy" remains not greater than 15%.

Cell viability assays of the "near-pure zinc alloy" show survival rates not less than 80% for human aortic smooth muscle cells (HASMCs), fibroblasts (human aortic adventitial fibroblasts, HAAFs), endothelial cells (human umbilical vein endothelial cells, HUVECs), and murine pre-osteoblasts (MC3T3-E1). Antibacterial efficacy against Escherichia coli (*E. coli*) and Staphylococcus aureus (*S. aureus*) reaches not less than 90%.

A second aspect of the present disclosure is to provide a preparation method of the high-performance near-pure zinc alloy.

The preparation method of the high-performance near-pure zinc alloy includes: melting→heat treatment→ severe plastic deformation→rapid cooling.

The method combining severe plastic deformation with rapid cooling induces the formation of abundant defects (e.g., vacancies, grain boundaries, twin boundaries), which promotes the establishment of co-segregation structures by multiple alloying elements at defect sites such as grain boundaries and twin boundaries.

Preferably, a melting process of the high-performance near-pure zinc alloy utilizes pure metals, corresponding to the elements within the final product, as raw materials; For instance, the zinc raw material used is pure zinc with a purity greater than 99.99; and the melting process is conducted 3 to 10 times under vacuum or inert gas protection to obtain an ingot of the high-performance near-pure zinc alloy. This can make the chemical composition more uniform and reduce the formation of casting defects.

The melting conditions are as follows: near-pure zinc alloy is melted in ceramic crucibles such as $Al_2O_3$ with a melting point above 2000°C. The crucible material is selected according to the principle that its melting point must be at least 800°C higher than that of pure zinc (i.e., 419.5°C), thereby preventing chemical contamination of the near-pure zinc alloy of the present disclosure by the crucible material. The melting process is conducted in the following two steps: (i) heating to a temperature of 480°C to 550°C and holding for 1 min to 3 min; (ii) cooling to a temperature of 420°C to 460°C and holding for 1 min to 5 min. This two-step melting process promotes co-segregation of the alloy elements at Zn grain boundaries.

The electromagnetic stirring is applied during the melting process, with a frequency of 800 Hz to 3,000 Hz, thus making the melt uniform; and at least one melt covering agent selected from the group consisting of KCl, NaCl, $MgCl_2$, LiCl, and borax is added during the melting process.

The heat treatment is at least one selected from the group consisting of homogenization heat treatment, solution heat treatment, aging heat treatment, and two-step aging heat treatment.

Preferably, the heat treatment includes, but is not limited to, the following four treatment modes:

(1) homogenization heat treatment;

(2) solution heat treatment;

(3) conducting the solution heat treatment and aging heat treatment in sequence; and

(4) conducting the solution heat treatment and two-step aging heat treatment in sequence.

Preferably, the homogenization heat treatment is conducted at 200°C to 360°C for 3 h to 10 h; the solution heat treatment is conducted at 300°C to 360°C for 8 h to 20 h; the aging heat treatment is conducted at 50°C to 180°C for 0.1 h to 12 h.

The two-step aging heat treatment includes: a first-stage aging heat treatment conducted at 50°C to 80°C for 5 min to 6 h and a second-stage aging heat treatment conducted at 100°C to 150°C for 5 min to 10 h.

A cooling method after the heat treatment is any one selected from the group consisting of air cooling, furnace cooling, water quenching, and oil quenching; the severe plastic deformation is at least one selected from the group consisting of grooved rolling, rolling, equal channel angular pressing (ECAP), high-pressure torsion (HPT), and high-velocity impact; the severe plastic deformation is conducted at -50°C to 320°C, with heat preservation for 15 min to 3 h before deformation; and the severe plastic deformation has a single-pass deformation greater than or equal to 20% and a total deformation greater than or equal to 98%; the grooved rolling is conducted at a rolling speed of 0.05 m/s to 0.6 m/s, with a single-pass deformation of 20% to 28% and a total deformation of 98% to 99%; the rolling is conducted at a rolling speed of 0.5 m/s to 2.5 m/s, with a single-pass deformation of 30% to 45% and a total deformation of 98% to 99.5%; the ECAP is conducted at an extrusion speed of 1 mm/s to 20 mm/s, with a number of extrusion passes of 10 to 25 and a total deformation of 98% to 99.7%; the HPT is conducted under an applied pressure of 0.5 GPa to 6 GPa at a rotational speed of 0.3 r/min to 2 r/min, with a number of rotations of 2 to 6; and the high-velocity impact is conducted at an impact velocity of 10 m/s to 100 m/s under an impact load of 100 N to 1,000 N in an impact angle of 45° to 60° for 0.1 s to 1 s.

The rapid cooling after the severe plastic deformation is quenching in any one selected from the group consisting of a water bath, an oil bath, and liquid nitrogen.

A third aspect of the present disclosure is to provide use of the high-performance "near-pure zinc alloy" prepared by the preparation method.

In the present disclosure, the near-pure zinc alloy is suitable for manufacturing the following seven kinds of products: (1) biodegradable stents, including cardiac coronary and other vascular stents, urethral stents, biliary stents, tracheal stents, esophageal stents, intestinal stents, porous bone-defect-filling scaffolds, and bone tissue repair scaffolds; (2) bone implant devices such as bone nails, bone pins, bone plates, suture anchors, screws, bone sleeves, bone fixators, and compression plates; (3) surgical instruments including intestinal/vascular/neural anastomotic staplers or staples, and vascular clips; (4) biomedical membranes encompassing guided bone regeneration (GBR) membranes, guided tissue regeneration (GTR) membranes, artificial heart valves, and meningeal repair membranes; (5) industrial components including dissolvable bridge plugs and dissolvable frac balls for petroleum drilling; (6) zinc alloy die-cast parts such as shell-type components, wear-resistant and damping parts; (7) electrogalvanized zinc materials, construction zinc materials, battery-grade zinc materials, printing-use zinc materials, and zinc-based alloy solders.

The technical key of the present disclosure lies in:

The critical innovation lies in overcoming conventional limitations: ordinary pure zinc undergoes plastic deformation with strains not greater than 85%, requiring neither severe plastic deformation nor rapid cooling, as these processes yield negligible performance improvements. In contrast, the present disclosure introduces trace alloying elements (total content not greater than 0.5 at.%) to pure zinc, resulting in near-pure zinc alloy. By applying severe plastic deformation followed by rapid cooling, two or more of the segregation-prone elements Mg, Mn, Li, Cu, Ag, Ca, Sr, Fe, Zr, Ti, Al, C, Ga and Si are driven to form co-segregation structures-rather than intermetallic second-phase particles-at crystal defects such as grain boundaries, sub-grain boundaries, twin boundaries and dislocations. This approach fundamentally transforms the near-pure zinc alloy's performance, delivering properties substantially superior to pure zinc. The near-pure zinc alloy achieves: yield strength (YS) not less than 250 MPa, ultimate tensile strength (UTS) not less than 320 MPa, elongation to failure (EL) not less than 25%, mechanical property variation after one-year room-temperature storage not greater than 5%, maximum bending force not less than 2 kN, stress corrosion susceptibility factor not greater than 15%, uniform degradation with post-60-d-immersion corrosion pit size (in equivalent diameter) not greater than 15 $\mu$m with standard deviation not greater than 3 $\mu$m, and uniformity coefficient ($\eta$) of pitting distribution not less than 80%. The co-segregation structure simultaneously enhances strength while maintaining superior mechanical stability, improves stress corrosion resistance and suppresses intergranular cracking, enhances plasticity via shifting plastic deformation mode from dislocation slip to grain boundary motion, and attenuates galvanic corrosion effects to promote uniform degradation.

Compared with the prior art, the present disclosure at least has the following six advantages:

1. The total content of alloying elements added to the near-pure zinc alloy is not greater than 0.5 at.%, while the Zn content still complies with the purity requirements for pure zinc specified by the Chinese National Standard GB/T 470-2008 (equivalent to ISO 752:2004 Zinc ingots). Crucially, the near-pure zinc alloy exhibits significantly higher strength and mechanical stability than pure zinc. Specifically, it delivers high performance under ultra-low alloying addition: YS not less than 250 MPa, UTS not less than 320 MPa, EL not less than 25%, mechanical property variation after one-year room-temperature storage not greater than 5%, maximum bending force not less than 2 kN, stress corrosion susceptibility factor not greater than 15%, uniform degradation with post-60-d-immersion corrosion pit size (in equivalent diameter) not greater than 15 $\mu$m with standard deviation not greater than 3 $\mu$m, and uniformity coefficient ($\eta$) of pitting distribution not less than 80%.

2. By applying severe plastic deformation followed by rapid cooling, two or more of the segregation-prone elements Mg, Mn, Li, Cu, Ag, Ca, Sr, Fe, Zr, Ti, Al, C, Ga and Si are driven to form co-segregation structures-rather than intermetallic second-phase particles-at crystal defects such as grain boundaries, sub-grain boundaries, twin boundaries and dislocations.

3. The co-segregation structure formed in the near-pure zinc alloy simultaneously enhances strength while maintaining superior mechanical stability, improves stress corrosion resistance and suppresses intergranular cracking, enhances plasticity via shifting plastic deformation mode from dislocation slip to grain boundary motion, and attenuates galvanic corrosion effects to promote uniform degradation.

4. In the near-pure zinc alloy of the present invention, the total amount of alloying elements is low; the volume fraction of second-phase particles is not greater than 2%, and their equivalent diameter is not greater than 1 $\mu$m. This effectively avoids the problems common in highly alloyed Zn alloys-such as delayed metabolism of alloying elements, long-term retention of second-phase particles and degradation products, and compromised biocompatibility.

5. The near-pure zinc alloy of the present invention exhibits an average Zn grain size not larger than 2 $\mu$m and a dislocation density not greater than $2 \times 10^{14}$ m$^{-2}$. After holding in air at 37-200°C for 5-100 h, the average Zn grain size remains not larger than 10 $\mu$m, demonstrating excellent microstructural stability. Moreover, its recrystallization temperature is not lower than 80°C, markedly higher than that of pure zinc (=15°C).

6. The preparation method facilitates industrial-scale mass production.

## BRIEF DESCRIPTION OF THE DRAWINGS

In order to describe the technical solutions in the examples of the present disclosure more clearly, the accompanying drawings required to describe the examples are briefly described below. Apparently, the accompanying drawings described below are only some examples of the present disclosure. Those of ordinary skill in the art may further obtain other accompanying drawings based on these accompanying drawings without inventive effort.

FIG. 1 shows the co-segregation structure of Mn and Mg elements at grain boundaries in near-pure zinc alloy 2 prepared according to Example 2 of the present disclosure (ellipse-marked areas indicating co-segregation zones);

FIG. 2 shows the zinc grain microstructure of near-pure zinc alloy 5 prepared according to Example 5 of the present disclosure;

FIG. 3 shows the tensile mechanical property bar chart of near-pure zinc alloy 2 prepared according to Example 2 of the present disclosure;

FIG. 4 shows the comparison diagram of maximum bending force between Example 8 of the present disclosure and pure zinc of the comparative example; and

FIG. 5 shows the corrosion pit morphology of the near-pure zinc alloy 10 prepared in Example 10 of the present disclosure after 60 days of immersion in 37 °C simulated body fluid (SBF) and subsequent removal of corrosion products.

## DETAILED DESCRIPTION OF THE EMBODIMENTS

To make the to-be-solved technical problems, technical solutions, and advantages of the present disclosure clearer, a detailed description is given below in conjunction with the accompanying drawings and specific embodiments.

Test Method:

The near-pure zinc alloy undergoes evaluations for recrystallization temperature, room-temperature tensile properties, room-temperature bending performance, corrosion rate, biocompatibility, and stress corrosion susceptibility.
Recrystallization temperature is determined via a dual-method approach combining metallographic analysis and hardness measurement;

Room-temperature tensile property is assessed according to ISO 6892-1:2009 "Metallic Materials - Tensile Testing - Part 1: Method of Test at Room Temperature";

Room-temperature bending performance is evaluated per ISO 14125:1998 "Metallic Materials - Bend Testing - Part 5: Three-Point Bend Test Method at Room Temperature";

Corrosion rate is measured following ASTM G31-72 "Standard Guide for Laboratory Immersion Corrosion Testing of Metals";

Biocompatibility is characterized in compliance with ISO 10993-5:2009 "Biological Evaluation of Medical Devices - Part 5: Tests for *In Vitro* Cytotoxicity"; and

Stress corrosion susceptibility is analyzed as per ISO 7539-1:2012 "Corrosion of metals and alloys - Stress corrosion testing - Part 1: General guidance on testing procedure".

**Examples 1 to 15**

Composition, preparation, and performance testing of high-performance ternary near-pure zinc alloys were conducted.

The chemical compositions of 15 examples of the ternary near-pure zinc alloys were presented in Table 1-1. The preparation process included: melting → homogenization heat treatment → grooved rolling → water quenching.

The batches were prepared according to the compositions of Alloys 1-15 listed in Table 1-1, and the zinc raw material used was pure zinc with a purity greater than 99.99%. Under argon protection, the raw materials were repeatedly melted 5 times in an induction furnace to obtain the corresponding near-pure zinc alloy ingots. The near-pure zinc alloy was melted in ceramic crucibles such as $Al_2O_3$ with a melting point above 2000°C. The crucible material was selected according to the principle that its melting point must be at least 800°C higher than that of pure zinc (i.e., 419.5°C), thereby preventing chemical contamination of the near-pure zinc alloy of the present disclosure by the crucible material. Melting proceeded in 2 steps: (i) heating to 500°C and holding for 1 min; (ii) cooling to 420°C and holding for 1 min. This two-step melting process promoted co-segregation of the alloy elements at Zn grain boundaries, after which the alloy melt was poured into a mold and cooled to room temperature.

Electromagnetic stirring at 900 Hz was applied during melting, which enhanced the purity and quality of the near-pure zinc alloy ingots. KCl melt covering agent was added to reduce metal oxidation and impurity formation, further improving purity of the near-pure zinc alloy ingots.

Homogenization heat treatment was performed at 360°C for 6 h followed by furnace cooling.

Grooved rolling was performed at 200°C with a pre- heating holding time of 0.5 h. Rolling speed was maintained at 0.2 m/s with single-pass deformation of 25% to 30% and total deformation of 98%. Alloys 1 to 15 in Table 1-1 exhibited excellent formability, eliminating the need for inter-pass annealing. The resulting rolled wires displayed satisfactory surface quality without cracks. In contrast, surface cracks appeared on the Zn-0.4Li and Zn-0.8Li alloy wires prepared by the same method.

Immediately water-quenched after groove rolling. This facilitated co-segregation of alloying elements at Zn grain boundaries while suppressing grain growth and preventing strength reduction.

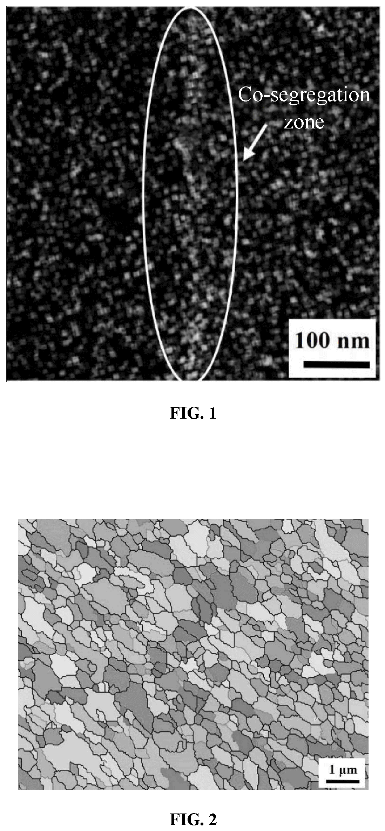

Transmission electron microscopy (TEM) analysis of Alloys 1 to 15 in Table 1-1 consistently revealed co-segregation of added elements at Zn grain boundaries. FIG. 1 showed the TEM microstructure of co-segregated Mn and Mg elements at Zn grain boundaries in Alloy 2. Comparative alloys Zn-0.6Mn, Zn-0.8Mn and Zn-1Mn prepared by the same method exhibited no grain boundary segregation.

Electron backscatter diffraction (EBSD) characterization of Alloys 1 to 15 in Table 1-1 demonstrated: the average size of Zn grains was not greater than 1.5 $\mu$m; volume fraction of second-phase particle was not greater than 1.6%; size of second-phase particle (in equivalent diameter) was not greater than 1 $\mu$m; dislocation density was not greater than $1.5 \times 10^{14}$ m$^{-2}$. After annealing for 10 h in air at 100°C, Alloys 1 to 15 maintained average sizes of Zn grains not greater than 2 $\mu$m, confirming exceptional microstructural stability. FIG. 2 showed Zn grains of Alloy 5 in Table 1-1.

Recrystallization temperatures of Alloys 1 to 15 in Table 1-1 ranged from 80°C to 120°C. Comparative pure zinc prepared by the same method exhibited recrystallization temperatures of 15°C to 25°C, confirming that trace elements in Alloys 1 to 15 substantially elevated recrystallization temperature of the zinc matrix.

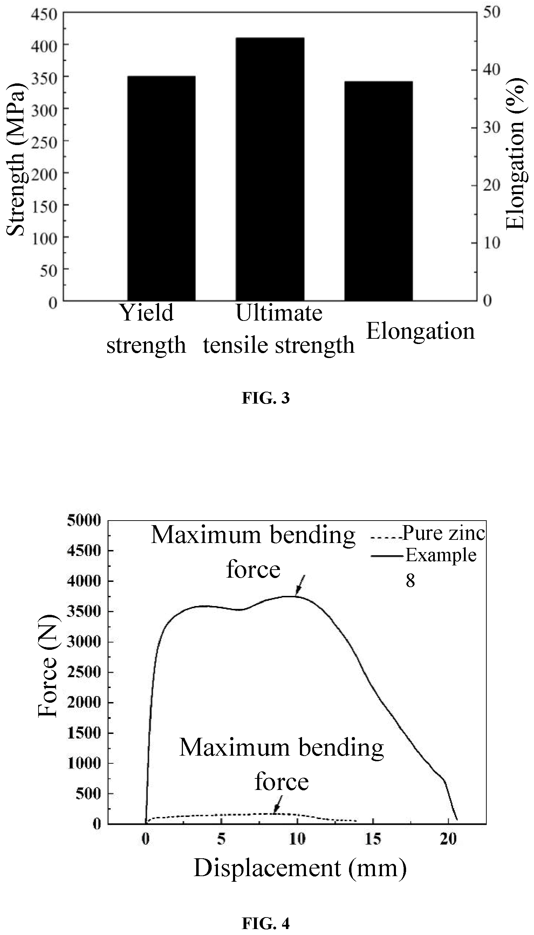

The room-temperature tensile properties of Alloys 1-15 in Table 1-1 were measured as follows: YS of 250-405 MPa, UTS of 320-500 MPa, EL of 25%-156%; after one-year storage in 37°C air: strength reduction was not greater than 3%, and elongation reduction was not greater than 5%. The room-temperature tensile properties of the comparative pure zinc prepared by the same method was: YS of 30-50 MPa, UTS of 80-120 MPa, EL of 5%-35%; after one-year storage in 37°C air: strength reduction was not less than 40%. These results demonstrated that the co-segregation structures at Zn grain boundaries significantly enhanced mechanical properties and stability in Alloys 1-15 in Table 1-1. FIG. 3 showed tensile properties of Alloy 2.

The maximum bending force of each alloy in Alloys 1-15 listed in Table 1-1 was measured to be 2.3-5 kN. However, those of pure Zn, Zn-1Mn, Zn-1.5Cu, Zn-0.6Fe, and Zn-0.6Ca alloys prepared by the same method were not greater than 0.8 kN. FIG. 4 presented a comparison of the maximum bending forces between Alloy 8 and the comparative pure zinc.

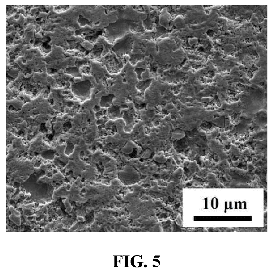

Degradation behavior in 37°C SBF: Alloys 1-15 in Table 1-1 exhibited uniform degradation; post-60-d immersion: corrosion pit size (in equivalent diameter) was not greater than 8 μm, standard deviation was not greater than 1.2 μm, uniformity coefficient (η) of pitting distribution was not less than 89%, and the degradation rate ranged from 0.02 to 0.5 mm/year. In contrast, comparative alloys prepared by the same method (pure zinc, Zn-0.6Mn, Zn-0.8Mn, Zn-1Mn, Zn-1Cu, Zn-2Cu, Zn-0.8Fe, Zn-0.8Ca) exhibited localized corrosion in 37°C SBF; post-60-d immersion: corrosion pit size (in equivalent diameter) was not less than 35 μm, standard deviation was not less than 15 μm, uniformity coefficient (η) of pitting distribution was not greater than 50%. FIG. 5 displayed corrosion pit morphology of Alloy 10 after removing corrosion products post-60 d-immersion in 37°C SBF.

The stress corrosion susceptibility factors of Alloys 1-15 in Table 1-1 were measured to be not greater than 10%, whereas those of pure zinc, Zn-0.6Mn, Zn-0.8Mn, Zn-1Mn, Zn-1Cu, Zn-2Cu, Zn-0.8Fe, and Zn-0.8Ca alloys prepared by the same method were not less than 20%.

Biological performance of Alloys 1-15 in Table 1-1: cell viability values were not less than 80% for HASMCs, HUVECs, and MC3T3-E1 cells; antibacterial rates against *E. coli* and *S. aureus* were not less than 90%.

Table 1-2 summarized key properties of selected near-pure zinc alloy examples and comparative materials.

Table 1-1

| Example | Alloy composition (at.%) |
|---|---|
| Example 1 | Zn-0.006Mn-0.008Mg (referred to 0.006 at.% Mn and 0.008 at.% Mg, and the following alloy compositions were also expressed similarly and would not be described in detail later) |
| Example 2 | Zn-0.03Mg-0.005Mn |
| Example 3 | Zn-0.05Cu-0.1Mg |
| Example 4 | Zn-0.3Mn-0.2Ag |
| Example 5 | Zn-0.05Li-0.002Mn |
| Example 6 | Zn-0.2Ca-0.06Mg |
| Example 7 | Zn-0.45Li-0.05Si |
| Example 8 | Zn-0.3Mn-0.18Li |
| Example 9 | Zn-0.4Ga-0.08Al |
| Example 10 | Zn-0.3Ti-0.005Mg |
| Example 11 | Zn-0.2C-0.05Zr |
| Example 12 | Zn-0.3Al-0.1Sr |
| Example 13 | Zn-0.2Sr-0.1Ca |
| Example 14 | Zn-0.35Cu-0.02Fe |
| Example 15 | Zn-0.4Fe-0.08C |

Table 1-2

| | Near-pure zinc alloy/pure zinc | Total alloying elements/at.% | Whether co-segregation occurred | YS/MPa | UTS/MPa | EL/% | Mechanical property variation after 1-year room-temperature storage/% | Stress corrosion susceptibility factor/% | Post-60-d-immersion corrosion pit size/$\mu$m |
|---|---|---|---|---|---|---|---|---|---|
| Example | Alloy 2 | 0.08 | Yes | 350 | 410 | 38 | $\leq 2$ | $\leq 7$ | $\leq 6$ |
| | Alloy 4 | 0.5 | Yes | 260 | 326 | 150 | $\leq 3$ | $\leq 9$ | $\leq 8$ |
| | Alloy 7 | 0.5 | Yes | 400 | 490 | 30 | $\leq 1$ | $\leq 4$ | $\leq 4$ |
| | Alloy 8 | 0.48 | Yes | 402 | 481 | 66 | $\leq 2$ | $\leq 5$ | $\leq 6$ |
| | Alloy 10 | 0.305 | Yes | 386 | 460 | 52 | $\leq 3$ | $\leq 5$ | $\leq 5$ |
| Comparative Example | Pure zinc | 0 | No | 40 | 102 | 34 | $\geq 30$ | $\geq 48$ | $\geq 35$ |
| | Zn-1Mn | 1 | No | 140 | 187 | 56 | $\geq 15$ | $\geq 32$ | $\geq 46$ |

## Examples 16 and 30

Composition, preparation, and performance testing of high-performance quaternary near-pure zinc alloys were conducted.

The chemical compositions of 15 examples of the quaternary near-pure zinc alloys were presented in Table 2-1. The preparation process included: melting → solution heat treatment → grooved rolling → water quenching → rolling → oil quenching.

The batches were prepared according to the compositions of Examples 16-30 listed in Table 2-1, and the zinc raw material used was pure zinc with a purity greater than 99.99%. Under argon protection, the raw materials were repeatedly melted 6 times in an induction furnace to obtain the corresponding near-pure zinc alloy ingots. The near-pure zinc alloy was melted in ceramic crucibles such as $Al_2O_3$ with a melting point above 2000°C. The crucible material was selected according to the principle that its melting point must be at least 800°C higher than that of pure zinc (i.e., 419.5°C), thereby preventing chemical contamination of the near-pure zinc alloy of the present disclosure by the crucible material. Melting proceeded in 2 steps: (i) heating to 510°C and holding for 2 min; (ii) cooling to 430°C and holding for 1 min. This two-step melting process promoted co-segregation of the alloy elements at the Zn grain boundaries, after which the alloy melt was poured into a mold and cooled to room temperature.

Electromagnetic stirring at 1,000 Hz was applied during melting, which enhanced the purity and quality of the near-pure zinc alloy ingot. NaCl melt covering agent was added to reduce metal oxidation and impurity formation, further improving the purity of the near-pure zinc alloy ingots.

Solution heat treatment was performed at 360°C for 16 h followed by water quenching.

Grooved rolling was performed at 230 °C with a pre-heating holding time of 1 h. Rolling proceeded at 0.3 m/s with single-pass deformation of 28% to 35% and total deformation of 98%. Alloys 16 to 30 in Table 2-1 exhibited excellent formability, eliminating inter-pass annealing. Resulting rolled wires showed crack-free surfaces. In contrast, surface cracks appeared on the Zn-0.8Ca and Zn-1Fe alloy wires prepared by the same method.

Immediately water-quenched after groove rolling. This facilitated co-segregation of alloying elements at Zn grain boundaries while suppressing grain growth and preventing strength reduction.

Rolling was then conducted at 200 °C with a pre-heating holding time of 0.5 h. Rolling speed was 1 m/s with single-pass deformation of 32% to 37% and total deformation of 99%. Oil quenching was performed immediately after rolling.

TEM analysis confirmed co-segregation at Zn grain boundaries in all alloys in Table 2-1. Comparative alloys Zn-0.6Ag, Zn-0.8Ag, and Zn-1Ag showed no grain boundary segregation.

EBSD characterization of Alloys 16 to 30 in Table 2-1 demonstrated: the average size of Zn grains was not greater than 1.2 $\mu$m; volume fraction of second-phase particle was not greater than 2%; size of second-phase particle (in equivalent diameter) was not greater than 1 $\mu$m; dislocation density was not greater than $1.5 \times 10^{14}$ m$^{-2}$. After annealing for

10 h in air at 120°C, average sizes of Zn grains remained not greater than 4 $\mu$m, confirming microstructural stability.

Recrystallization temperatures of Alloys 16 to 30 in Table 2-1 measured from 86°C to 130°C. Comparative pure zinc prepared by the same method exhibited recrystallization temperatures of 15°C to 26°C, confirming that trace elements in Alloys 16 to 30 substantially elevated recrystallization temperature of the zinc matrix.

The room-temperature tensile properties of Alloys 16-30 in Table 2-1 were measured as follows: YS of 270-420 MPa, UTS of 340-530 MPa, EL of 28%-161%; post-one-year storage at 37°C: strength reduction was not greater than 2%, and elongation reduction was not greater than 4%. The room-temperature tensile properties of the comparative pure zinc prepared by the same method were: YS of 30-45 MPa, UTS of 68-110 MPa, EL of 5%-30%; post-one-year storage at 37°C: strength reduction was not less than 46%, elongation reduction was not less than 35%. These results demonstrated that the co-segregation structures at Zn grain boundaries significantly enhanced mechanical properties and stability in Alloys 16-30 in Table 2-1.

The maximum bending force of each alloy in Alloys 16-30 listed in Table 2-1 was measured to be 2.5-5 kN. However, those of pure Zn, Zn-1Mn, Zn-1Cu, and Zn-1Fe alloys prepared by the same method were not greater than 0.7 kN.

Degradation behavior in 37°C SBF: Alloys 16-30 in Table 2-1 exhibited uniform degradation; post-60-d immersion: corrosion pit size (in equivalent diameter) was not greater than 12 $\mu$m, standard deviation was not greater than 1.2 $\mu$m, and the degradation rate ranged from 0.04 to 0.6 mm/year. In contrast, comparative alloys prepared by the same method (pure zinc, Zn-0.7Mn, Zn-1Mn, Zn-2Cu, Zn-3Cu, Zn-0.6Fe, Zn-0.6Ca) exhibited localized corrosion in 37°C SBF; post-60-d immersion: corrosion pit size (in equivalent diameter) was not less than 40 $\mu$m, standard deviation was not less than 18 $\mu$m, uniformity coefficient ($\eta$) of pitting distribution was not greater than 45%.

The stress corrosion susceptibility factors of Alloys 16-30 in Table 2-1 were measured to be not greater than 8%, whereas those of pure zinc, Zn-0.6Ag, Zn-0.8Mn, Zn-1Mn, Zn-0.6Fe, and Zn-0.8Cu alloys prepared by the same method were not less than 25%.

Biological performance of Alloys 16-30 in Table 2-1: cell viability values were not less than 80% for HASMCs, HUVECs, and MC3T3-E1 cells; antibacterial rates against *E. coli* and *S. aureus* were not less than 90%.

Table 2-2 summarized key properties of selected near-pure zinc alloy examples and comparative materials.

Table 2-1

| Example | Alloy composition (at.%) |
|---|---|
| Example 16 | Zn-0.4Li-0.05Mn-0.03Mg |
| Example 17 | Zn-0.25Mg-0.05Li-0.008Mn |
| Example 18 | Zn-0.25Mn-0.1Mg-0.006Li |
| Example 19 | Zn-0.3Li-0.05Ca-0.004Mg |
| Example 20 | Zn-0.3Al-0.1Mg-0.08Ca |
| Example 21 | Zn-0.2C-0.2Ga-0.06Ti |
| Example 22 | Zn-0.1Si-0.1Ca-0.15Ti |
| Example 23 | Zn-0.4Li-0.05Sr-0.05Mn |
| Example 24 | Zn-0.3Cu-0.1Mg-0.02Fe |
| Example 25 | Zn-0.1Ag-0.1Ca-0.1Zr |
| Example 26 | Zn-0.2Mg-0.15Ca-0.02Cu |
| Example 27 | Zn-0.15Zr-0.1Ca-0.05Sr |
| Example 28 | Zn-0.15Fe-0.05Ti-0.008Cu |
| Example 29 | Zn-0.45Mg-0.02Ca-0.02Ga |
| Example 30 | Zn-0.15Sr-0.12C-0.02Si |

Table 2-2

| | Near-pure zinc alloy/pure zinc | Whether co-segregation occurred | YS/MPa | UTS/MPa | EL/% | Mechanical property variation after 1-year room-temperature storage/% | Stress corrosion susceptibility factor/% | Post-60-d-immersion corrosion pit size/$\mu$m |
|---|---|---|---|---|---|---|---|---|
| Example | Alloy 16 | Yes | 400 | 500 | 40 | ≤2 | ≤6 | ≤7 |
| | Alloy 23 | Yes | 418 | 480 | 70 | ≤1 | ≤3 | ≤8 |
| | Alloy 25 | Yes | 410 | 508 | 29 | ≤2 | ≤4 | ≤9 |
| | Alloy 29 | Yes | 420 | 528 | 30 | ≤1 | ≤2 | ≤7 |
| Comparative Example | Pure zinc | No | 36 | 80 | 30 | ≥35 | ≥50 | ≥40 |
| | Zn-1Fe | No | 86 | 141 | 8 | ≥26 | ≥36 | ≥52 |

**Examples 31 and 45**

Composition, preparation, and performance testing of high-performance pentanary near-pure zinc alloys were conducted.

The chemical compositions of 15 examples of the pentanary near-pure zinc alloys were presented in Table 3-1. The preparation process included: melting → solution heat treatment → aging heat treatment → HPT → water quenching.

The batches were prepared according to the compositions of Alloys 31-45 listed in Table 3-1, and the zinc raw material used was pure zinc with a purity greater than 99.99%. Under argon protection, the raw materials were repeatedly melted 7 times in an induction furnace to obtain the corresponding near-pure zinc alloy ingots. The near-pure zinc alloy was melted in ceramic crucibles such as $Al_2O_3$ with a melting point above 2000°C. The crucible material was selected according to the principle that its melting point must be at least 800°C higher than that of pure zinc (i.e., 419.5°C), thereby preventing chemical contamination of the near-pure zinc alloy of the present disclosure by the crucible material. Melting proceeded in 2 steps: (i) heating to 510°C and holding for 1 min; (ii) cooling to 430°C and holding for 2 min. This two-step melting process promoted co-segregation of the alloy elements at Zn grain boundaries, after which the alloy melt was poured into a mold and cooled to room temperature.

Electromagnetic stirring at 1,200 Hz was applied during melting, which enhanced the purity and quality of the near-pure zinc alloy ingots. $MgCl_2$ melt covering agent was added to reduce metal oxidation and impurity formation, further improving the purity of the near-pure zinc alloy ingots.

Solution heat treatment was performed at 360°C for 20 h followed by water quenching.

Aging heat treatment was conducted at 100°C for 0.1-6 h.

HPT processing occurred at 260°C after 30-min preheating, with applied pressure of 2 GPa, 3 rotations, and rotational speed of 1 rpm.

Immediate water quenching post-HPT facilitated co-segregation of alloying elements at Zn grain boundaries while suppressing grain growth and strength loss.

TEM analysis confirmed co-segregation at Zn grain boundaries in all Alloys 31-45 listed in Table 3-1. Comparative alloys Zn-0.6Ag, Zn-0.8Ca, and Zn-1Mn exhibited no grain boundary segregation.

EBSD characterization of Alloys 31 to 45 in Table 3-1 demonstrated: the average size of Zn grains was not greater than 1 $\mu$m; volume fraction of second-phase was not greater than 2%; size of second-phase (in equivalent diameter) was not greater than 1 $\mu$m; dislocation density was not greater than $1.8 \times 10^{14}$ m$^{-2}$. After annealing for 15 h in air at 160°C, average sizes of Zn grains remained not greater than 3 $\mu$m, confirming microstructural stability.

Recrystallization temperatures of Alloys 31 to 45 in Table 3-1 ranged from 95°C to 138°C. Comparative pure zinc prepared by the same method exhibited recrystallization temperatures of 15°C to 28°C, confirming trace elements in Alloys 31-45 substantially elevated recrystallization temperature of the zinc matrix.

The room-temperature tensile properties of Alloys 31-45 in Table 3-1 were measured as follows: YS of 280-430 MPa, UTS of 350-550 MPa, EL of 25%-148%; post-one-year storage at 37°C: strength reduction was not greater than 2%, and elongation reduction was not greater than 5%. The room-temperature tensile properties of the comparative pure zinc prepared by the same method were: YS of 40-48 MPa, UTS of 70-115 MPa, EL of 2%-30%; post-one-year storage at 37°C: strength reduction was not less than 45%, elongation reduction was not less than 33%. These results demonstrated that the co-segregation structures at Zn grain boundaries significantly enhanced mechanical properties and stability of in Alloys 31-45 in Table 3-1.

The maximum bending force of each alloy in Alloys 31-45 listed in Table 3-1 was measured to be 2.6-5.3 kN. However, those of pure Zn, Zn-0.8Mn, Zn-1Cu, Zn-1Ca, Zn-0.6Ga, and Zn-0.6Al alloys prepared by the same method were not greater than 0.7 kN.

Degradation behavior in 37°C SBF: Alloys 31-45 in Table 3-1: uniform degradation; post-60-d immersion: corrosion pit size (in equivalent diameter) was not greater than 13 $\mu$m, standard deviation was not greater than 1.8 $\mu$m, and the degradation rate ranged from 0.07 to 0.8 mm/year. In contrast, comparative alloys prepared by the same method (pure zinc, Zn-1Mn, Zn-1Zr, Zn-0.8Sr, Zn-0.8Cu, Zn-0.8Ag) exhibited localized corrosion in 37°C SBF; post-60-d immersion: corrosion pit size (in equivalent diameter) was not less than 42 $\mu$m, standard deviation was not less than 20 $\mu$m, uniformity coefficient ($\eta$) of pitting distribution was not greater than 40%.

The stress corrosion susceptibility factors of Alloys 31-45 in Table 3-1 were measured to be not greater than 7%, whereas those of pure zinc, Zn-1Mn, Zn-1Zr, Zn-0.8Sr, Zn-0.8Cu, Zn-0.8Ag, and Zn-1Ca alloys prepared by the same method were not less than 40%.

Biological performance of Alloys 31-45 in Table 3-1: cell viability values were not less than 80% for HASMCs, HUVECs, and MC3T3-E1 cells; antibacterial rates against *E. coli* and *S. aureus* were not less than 90%.

Table 3-2 summarized key properties of selected near-pure zinc alloy examples and comparative materials.

Table 3-1

| Example | Alloy composition (at.%) |
|---|---|
| Example 31 | Zn-0.08 Ag-0.05 Li-0.05 Mn-0.03Mg |
| Example 32 | Zn-0.1Mg-0.05Ca-0.05Sr-0.008Mn |
| Example 33 | Zn-0.1Mn-0.1Li-0.05Zr-0.002Ti |
| Example 34 | Zn-0.2Li-0.15Cu-0.05Mg-0.01Ca |
| Example 35 | Zn-0.3Cu-0.05Mg-0.05Ca-0.02Fe |
| Example 36 | Zn-0.1Ag-0.1Mn-0.1Li-0.04Ti |
| Example 37 | Zn-0.1Mn-0.08Ti-0.08Ca-0.06Mg |
| Example 38 | Zn-0.3Al-0.05Sr-0.05Mg-0.02Zr |
| Example 39 | Zn-0.1Cu-0.1Mg-0.05Ca-0.05Fe |
| Example 40 | Zn-0.3Ag-0.1Ca-0.05Zr-0.02Sr |
| Example 41 | Zn-0.2Mg-0.2Cu-0.05Ca-0.02Fe |
| Example 42 | Zn-0.15Zr-0.1Ca-0.05Sr-0.05Ga |
| Example 43 | Zn-0.15Fe-0.1Cu-0.08Ca-0.05Ti |
| Example 44 | Zn-0.3Mg-0.1Li-0.05Ca-0.02Ti |
| Example 45 | Zn-0.1C-0.1Zr-0.05Sr-0.03Ca |

Table 3-2

| | Near-pure zinc alloy/pure zinc | Whether co-segregation occurred | YS/MPa | UTS/MPa | EL/% | Mechanical property variation after 1-year room-temperature storage/% | Stress corrosion susceptibility factor/% | Post-60-d-immersion corrosion pit size/$\mu$m |
|---|---|---|---|---|---|---|---|---|
| Example | Alloy 34 | Yes | 380 | 465 | 74 | $\leq$2 | $\leq$6 | $\leq$12 |
| | Alloy 38 | Yes | 415 | 510 | 32 | $\leq$2 | $\leq$6 | $\leq$9 |
| | Alloy 41 | Yes | 423 | 523 | 28 | $\leq$1 | $\leq$5 | $\leq$10 |
| | Alloy 44 | Yes | 427 | 541 | 26 | $\leq$1 | $\leq$4 | $\leq$11 |

(continued)

| | Near-pure zinc alloy/pure zinc | Whether co-segregation occurred | YS/MPa | UTS/MPa | EL/% | Mechanical property variation after 1-year room-temperature storage/% | Stress corrosion susceptibility factor/% | Post-60-dimmersion corrosion pit size/μm |
|---|---|---|---|---|---|---|---|---|
| Comparative Example | Pure zinc | No | 42 | 86 | 28 | ≥33 | ≥55 | ≥42 |
| | Zn-1C a | No | 102 | 189 | 5 | ≥26 | ≥40 | ≥65 |

**Examples 46 and 60**

Composition, preparation, and performance testing of high-performance multinary near-pure zinc alloys were conducted.

The chemical compositions of 15 examples of the multinary near-pure zinc alloys were presented in Table 4-1. The term "multinary" denoted alloys containing at least 6 elements. The preparation process included: melting → solution heat treatment → two-step aging heat treatment → ECAP → water quenching.

The batches were prepared according to the compositions of Examples 46-60 listed in Table 4-1, and the zinc raw material used was pure zinc with a purity greater than 99.99%. Under argon protection, the raw materials were repeatedly melted 8 times in an induction furnace to obtain the corresponding near-pure zinc alloy ingots. The near-pure zinc alloy was melted in ceramic crucibles such as $Al_2O_3$ with a melting point above 2000°C. The crucible material was selected according to the principle that its melting point must be at least 800°C higher than that of pure zinc (i.e., 419.5°C), thereby preventing chemical contamination of the near-pure zinc alloy of the present disclosure by the crucible material. Melting proceeded in 2 steps: (i) heating to 520°C and holding for 3 min; (ii) cooling to 420°C and holding for 3 min. This two-step melting process promoted co-segregation of the alloy elements at the Zn grain boundaries, after which the alloy melt was poured into a mold and cooled to room temperature.

Electromagnetic stirring at 1,500 Hz was applied during melting, which enhanced the purity and quality of the near-pure zinc alloy ingots. Borax melt covering agent was added to reduce metal oxidation and impurity formation, further improving the purity of the near-pure zinc alloy ingots.

Solution heat treatment was performed at 360°C for 20 h followed by water quenching.

Two-step aging heat treatment included: first-step aging heat treatment: 60°C for 1 h, second-step aging heat treatment: 100°C for 3 h.

ECAP processing occurred at 260°C with 15 to 25 passes, extrusion speed of 10 mm/s, and total deformation of 99.5%. Alloys 46 to 60 in Table 4-1 exhibited excellent formability, eliminating inter-pass annealing in ECAP. Resulting wires showed crack-free surfaces. In contrast, surface cracks appeared on the Zn-0.6Fe and Zn-0.8Sr alloy wires prepared by the same method.

Immediately water-quenched after ECAP. This facilitated co-segregation of alloying elements at Zn grain boundaries while suppressing grain growth and preventing strength reduction.

TEM analysis confirmed co-segregation of added elements at Zn grain boundaries in all alloys 46 to 60 in Table 4-1. Comparative alloys Zn-0.8Ca, Zn-0.8Mn, Zn-1Fe showed no grain boundary segregation.

EBSD characterization of Alloys 46 to 60 in Table 4-1 demonstrated: the average size of Zn grains was not greater than 1 μm; volume fraction of second-phase particle was not greater than 2%; size of second-phase particle (in equivalent diameter) was not greater than 1 μm; dislocation density was not greater than $1.9 \times 10^{14}$ m$^{-2}$. After annealing for 26 h in air at 200°C, the average size of Zn grains remained not greater than 3 μm, confirming microstructural stability.

Recrystallization temperatures of Alloys 46 to 60 in Table 4-1 measured from 100°C to 146°C. Comparative pure zinc prepared by the same method exhibited recrystallization temperatures of 15°C to 30°C, confirming that trace elements in Alloys 46 to 60 substantially elevated recrystallization temperature of the zinc matrix.

The room-temperature tensile properties of Alloys 46-60 in Table 4-1 were measured as follows: YS of 300-450 MPa, UTS of 380-580 MPa, EL of 25%-95%; post-one-year storage at 37°C: strength reduction was not greater than 2%, and elongation reduction was not greater than 5%. The room-temperature tensile properties of the comparative pure zinc prepared by the same method were: YS of 40-60 MPa, UTS of 70-130 MPa, EL of 5%-35%; post-one-year storage at 37°C: strength reduction was not less than 50%, elongation reduction was not less than 38%. These results demonstrated that the co-segregation structures at Zn grain boundaries significantly enhanced mechanical properties and stability in Alloys 46-60 in Table 4-1.

The maximum bending force of each alloy in Alloys 46-60 listed in Table 4-1 was measured to be 2.8-5.5 kN.

However, those of pure Zn, Zn-0.8Ca, Zn-1Fe, Zn-0.6Si, and Zn-0.6Al alloys prepared by the same method were not greater than 0.5 kN.

Degradation behavior in 37°C SBF: Alloys 46-60 in Table 4-1 exhibited uniform degradation; post-60-d immersion: corrosion pit size (in equivalent diameter) was not greater than 15 $\mu$m, standard deviation was not greater than 3 $\mu$m, and the degradation rate ranged from 0.07 to 1.5 mm/year; In contrast, comparative alloys prepared by the same method (pure zinc, Zn-0.6Ti, Zn-0.8Zr, Zn-1Mn, Zn-1Ga, Zn-2Al, Zn-0.8Si, Zn-0.8C) exhibited localized corrosion in 37°C SBF; post-60-d immersion: corrosion pit size (in equivalent diameter) was not less than 45 $\mu$m, standard deviation was not less than 30 $\mu$m, uniformity coefficient was not greater than 46%.

The stress corrosion susceptibility factors of Alloys 46-60 in Table 4-1 were measured to be not greater than 6%, whereas those of pure zinc, Zn-0.8Sr, Zn-1Mn, Zn-1Ga, Zn-5Al, Zn-1Si, and Zn-0.8C alloys prepared by the same method were not less than 42%.

Biological performance of Alloys 46 to 60 in Table 4-1: cell viability values were not less than 80% for HASMCs, HUVECs, and MC3T3-E1 cells; antibacterial rates against *E. coli* and *S. aureus* were not less than 90%.

Table 4-2 summarized key properties of selected near-pure zinc alloy examples and comparative materials.

Table 4-1

| Example | Alloy composition (at.%) |
|---|---|
| Example 46 | Zn-0.08Sr-0.05Ca-0.05Mn-0.03Mg-0.03Li |
| Example 47 | Zn-0.08Ti-0.05Mg-0.02Li-0.005Mn-0.002Sr |
| Example 48 | Zn-0.2Li-0.1Mg-0.08Cu-0.02Ti-0.02Mn-0.01Ca |
| Example 49 | Zn-0.3Mg-0.05Ti-0.04C-0.03Sr-0.03Zr-0.008Mn |
| Example 50 | Zn-0.05Ag-0.05Cu-0.05Mg-0.05Ca-0.05Zr-0.02Fe-0.03Li |
| Example 51 | Zn-0.1Mn-0.05Sr-0.03Mg-0.03Ti-0.03Zr-0.02Al-0.02Ag-0.006Ca-0.001Fe |
| Example 52 | Zn-0.15Li-0.05Sr-0.05Cu-0.04Mg-0.03Ti-0.03Zr-0.02Mn-0.02Ag-0.01Fe-0.01C a |
| Example 53 | Zn-0.3Cu-0.08Sr-0.04Mn-0.02Mg-0.03Zr-0.01Ag-0.005C-0.003Ca-0.003Ti-0.00 3Fe-0.001Li |
| Example 54 | Zn-0.15Zr-0.1Ag-0.1Al-0.05C-0.02Cu-0.02Ti-0.02Ca-0.006Si-0.003Mn-0.001M g |
| Example 55 | Zn-0.13Mn-0.1Ti-0.1C-0.06Fe-0.05Si-0.04Li -0.006Al-0.003Ag-0.002Zr-0.002Ga-0.001Sr |
| Example 56 | Zn-0.12Ga-0.08Mg-0.08Ca-0.06Mn-0.05Ag-0.04Li-0.03C-0.02Al-0.01Si-0.006 Ti-0.002Cu |
| Example 57 | Zn-0.17Mg-0.09Li-0.08Ti-0.06Zr-0.05Al-0.01Ga-0.008Mn-0.007Ca-0.006Ag-0. 006Sr-0.005Cu-0.005Fe-0.001C-0.005Si |
| Example 58 | Zn-0.2Mn-0.1Cu-0.08Si-0.06Ag-0.02Sr-0.01Fe-0.008Li-0.007Mg-0.005Ga-0.00 1Ca-0.001Al |
| Example 59 | Zn-0.12Ti-0.09Fe-0.08Mn-0.05Ca-0.05Al-0.05Sr-0.02Li-0.01Ga-0.009Cu-0.009 Ag-0.007Zr-0.003C-0.001Si |
| Example 60 | Zn-0.1Sr-0.08Cu-0.08Ag-0.07Zr-0.05Mg-0.05Ti-0.04Ca-0.04C-0.008Al-0.005M n-0.001Fe |

Table 4-2

| | Near-pure zinc alloy/pure zinc | Whether co-segregati on occurred | YS/M Pa | UTS/M Pa | EL/ % | Mechanical property variation after 1-year room-temperat ure storage/% | Stress corrosion susceptibili ty factor/% | Post-60-d-immers ion corrosion pit size/$\mu$m |
|---|---|---|---|---|---|---|---|---|
| Example | Alloy 46 | Yes | 436 | 530 | 36 | ≤2 | ≤5 | ≤12 |
| | Alloy 51 | Yes | 360 | 450 | 78 | ≤1 | ≤3 | ≤14 |
| | Alloy 57 | Yes | 430 | 550 | 31 | ≤1 | ≤2 | ≤15 |
| | Alloy 59 | Yes | 450 | 580 | 29 | ≤1 | ≤2 | ≤14 |

(continued)

| | Near-pure zinc alloy/pure zinc | Whether co-segregation occurred | YS/MPa | UTS/MPa | EL/% | Mechanical property variation after 1-year room-temperature storage/% | Stress corrosion susceptibility factor/% | Post-60-d-immersion corrosion pit size/$\mu$m |
|---|---|---|---|---|---|---|---|---|
| Comparative Example | Pure zinc | No | 50 | 96 | 30 | ≥38 | ≥58 | ≥45 |
| | Zn-0.6Ti | No | 120 | 196 | 5 | ≥30 | ≥42 | ≥53 |

## Claims

1. A high-performance near-pure zinc alloy, wherein
the high-performance near-pure zinc alloy, in atomic percent (at.%), has a zinc (Zn) content not less than 99.5%, with a balance being trace alloying elements intentionally added; the high-performance near-pure zinc alloy has a yield strength not less than 250 MPa, an ultimate tensile strength not less than 320 MPa, an elongation not less than 25%, a variation in mechanical properties after 1 year of room-temperature storage not greater than 5%, a maximum bending force not less than 2 kN, and a stress corrosion susceptibility factor not greater than 15%; and the high-performance near-pure zinc alloy exhibits high degradation uniformity, showing an average size, namely, in equivalent diameter of corrosion pits not greater than 15 $\mu$m after 60 d of immersion, with a standard deviation not greater than 3 $\mu$m and a uniformity coefficient of corrosion pit distribution not less than 80%.

2. The high-performance near-pure zinc alloy according to claim 1, wherein
the high-performance near-pure zinc alloy, in atomic percent (at.%), has a total content of the trace alloying elements intentionally added not greater than 0.5%, and comprises at least two elements selected from the following 14 elements: Mg, Mn, Li, Cu, Ag, Ca, Sr, Fe, Zr, Ti, Al, C, Ga, and Si.

3. The high-performance near-pure zinc alloy according to claim 1, wherein
second-phase particles in the high-performance near-pure zinc alloy have a volume fraction not greater than 2% and an average size, namely, in equivalent diameter not greater than 1 $\mu$m.

4. The high-performance near-pure zinc alloy according to claim 1, wherein
Zn grains in the high-performance near-pure zinc alloy have an average grain size not greater than 2 $\mu$m and a dislocation density not greater than $2\times10^{14}$ m$^{-2}$; and after exposure at 37°C to 200°C for 5 h to 100 h, the average size of the Zn grains is not greater than 10 $\mu$m.

5. A preparation method of the high-performance near-pure zinc alloy according to any one of claims 1 to 4, comprising the following steps: melting→heat treatment→severe plastic deformation→rapid cooling.

6. The high-performance near-pure zinc alloy and the preparation method according to any one of claims 1 to 5, wherein
the high-performance near-pure zinc alloy forms a co-segregation structure driven by two or more segregation-prone elements selected from the group consisting of Mg, Mn, Li, Cu, Ag, Ca, Sr, Fe, Zr, Ti, Al, C, Ga, and Si at crystal defects comprising grain boundaries, sub-grain boundaries, twin boundaries, and dislocation defects, rather than intermetallic second-phase particles.

7. The preparation method of the high-performance near-pure zinc alloy according to claim 5, wherein
a melting process of the high-performance near-pure zinc alloy adopts pure metals corresponding to constituent elements in the high-performance near-pure zinc alloy, metallic Zn has a purity greater than 99.99% as a raw material; and the melting process is conducted 3 to 10 times under vacuum or inert gas protection to obtain an ingot of the high-performance near-pure zinc alloy.

8. The preparation method of the high-performance near-pure zinc alloy according to claim 7, wherein
the melting process of the high-performance near-pure zinc alloy is conducted in a ceramic crucible comprising Al$_2$O$_3$ with a melting point greater than 2,000°C; a crucible material is a ceramic material with a melting point at least 800°C greater than a melting point of pure zinc; and the melting process is conducted in two steps comprising heating to a temperature of 480°C to 550°C and holding the temperature of 480°C to 550°C for 1 min to 3 min and then cooling to a temperature of 420°C to 460°C and holding the temperature of 420°C to 460°C for 1 min to 5 min.

9. The preparation method of the high-performance near-pure zinc alloy according to claim 7, wherein electromagnetic stirring is applied during the melting process, with a frequency of 800 Hz to 3,000 Hz; and at least one melt covering agent selected from the group consisting of KCl, NaCl, $MgCl_2$, LiCl, and borax is added during the melting process.

10. The preparation method of the high-performance near-pure zinc alloy according to claim 5, wherein the heat treatment is at least one selected from the group consisting of homogenization heat treatment, solution heat treatment, aging heat treatment, and duplex aging heat treatment.

11. The preparation method of the high-performance near-pure zinc alloy according to claim 5, wherein the heat treatment comprises, but is not limited to, the following four treatment modes:

(1) homogenization heat treatment;
(2) solution heat treatment;
(3) conducting the solution heat treatment and aging heat treatment in sequence; and
(4) conducting the solution heat treatment and duplex aging heat treatment in sequence.

12. The preparation method of the high-performance near-pure zinc alloy according to claim 11, wherein

the homogenization heat treatment is conducted at 200°C to 360°C for 3 h to 10 h;
the solution heat treatment is conducted at 300°C to 360°C for 8 h to 20 h;
the aging heat treatment is conducted at 50°C to 180°C for 0.1 h to 12 h;
the duplex aging heat treatment comprises: a first-stage aging heat treatment conducted at 50°C to 80°C for 5 min to 6 h and a second-stage aging heat treatment conducted at 100°C to 150°C for 5 min to 10 h; and
a cooling method after the heat treatment is any one selected from the group consisting of air cooling, furnace cooling, water quenching, and oil quenching.

13. The preparation method of the high-performance near-pure zinc alloy according to claim 5, wherein the severe plastic deformation is at least one selected from the group consisting of grooved rolling, rolling, equal channel angular pressing (ECAP), high-pressure torsion (HPT), and high-velocity impact.

14. The preparation method of the high-performance near-pure zinc alloy according to claim 5 or 13, wherein

the severe plastic deformation is conducted at -50°C to 320°C, with heat preservation for 15 min to 3 h before deformation; and
the severe plastic deformation has a single-pass deformation not less than 20% and a total deformation not less than 98%.

15. The preparation method of the high-performance near-pure zinc alloy according to claim 13, wherein

the grooved rolling is conducted at a rolling speed of 0.05 m/s to 0.6 m/s, with a single-pass deformation of 20% to 28% and a total deformation of 98% to 99%;
the rolling is conducted at a rolling speed of 0.5 m/s to 2.5 m/s, with a single-pass deformation of 30% to 45% and a total deformation of 98% to 99.5%;
the ECAP is conducted at an extrusion speed of 1 mm/s to 20 mm/s, with a number of extrusion passes of 10 to 25 and a total deformation of 98% to 99.7%;
the HPT is conducted under an applied pressure of 0.5 GPa to 6 GPa at a rotational speed of 0.3 r/min to 2 r/min, with a number of rotations of 2 to 6; and
the high-velocity impact is conducted at an impact velocity of 10 m/s to 100 m/s under an impact load of 100 N to 1,000 N in an impact angle of 45° to 60° for 0.1 s to 1 s.

16. The preparation method of the high-performance near-pure zinc alloy according to claim 5, wherein the rapid cooling after the severe plastic deformation is quenching in any one selected from the group consisting of a water bath, an oil bath, and liquid nitrogen.

17. Use of a high-performance near-pure zinc alloy prepared by the preparation method according to any one of claims 5 to 16 in manufacture of biodegradable stents, bone-implant devices, intestinal/vascular/neural anastomotic staplers or staples, vascular clips, guided bone-regeneration (GBR) membranes, guided tissue-regeneration (GTR) membranes, artificial heart valves, dura-mater repair membranes, dissolvable bridge plugs and dissolvable fracturing balls for petroleum drilling, zinc-alloy die castings, galvanized zinc materials, architectural zinc materials, battery-grade zinc materials, printing-grade zinc materials, and zinc-based brazing alloys.

**FIG. 1**

**FIG. 2**

**FIG. 3**

**FIG. 4**

**FIG. 5**

## INTERNATIONAL SEARCH REPORT

| International application No. |
| --- |
| **PCT/CN2024/137960** |

### A. CLASSIFICATION OF SUBJECT MATTER

C22C18/00(2006.01)i; C22C18/02(2006.01)i; C22C18/04(2006.01)i; C22C1/00(2023.01)n; C22F1/16(2006.01)i; C22F3/02(2006.01)n; C22F1/00(2006.01)i; A61L27/04(2006.01)i; A61L27/50(2006.01)i

According to International Patent Classification (IPC) or to both national classification and IPC

### B. FIELDS SEARCHED

Minimum documentation searched (classification system followed by classification symbols)

IPC:C22C C22F A61L

Documentation searched other than minimum documentation to the extent that such documents are included in the fields searched

Electronic data base consulted during the international search (name of data base and, where practicable, search terms used)

CNTXT, DWPI, CNKI, EPODOC, Elsevier Science: 变形量, 变形率, 大变形, 共偏聚, 共偏析, 加工率, 压下量, 压下率, 锌, Deformation, Deformation Rate, Large Deformation, Co-polarization, Co-segregation, Processing Rate, Reduction Rate, Zinc

### C. DOCUMENTS CONSIDERED TO BE RELEVANT

| Category* | Citation of document, with indication, where appropriate, of the relevant passages | Relevant to claim No. |
| --- | --- | --- |
| PX | CN 118389906 A (UNIVERSITY OF SCIENCE AND TECHNOLOGY BEIJING) 26 July 2024 (2024-07-26)<br>claims 1-17 | 1-17 |
| Y | HUANG, Zhifeng et al. "Solute-solute Interactions and their Impacts on Solute Co-segregation and Interfacial Cohesion of $\{10\bar{1}2\}$ Twin Boundary in Zinc"<br>*Journal of Materials Science & Technology,*<br>Vol. 138, No. 2023, 19 October 2022 (2022-10-19), pages 117-128<br>section 4, last two paragraphs, and section 5, conclusions | 1-17 |
| Y | WANG, L. Q. (WANG, Liqing) et al. "Effect of Cumulative Strain on the Microstructural and Mechanical Properties of Zn-0.02 wt%Mg Alloy Wires during Room-temperature Drawing Process"<br>*Journal of Alloys and Compounds,* Vol. 740, 05 March 2018 (2018-03-05), pages 949-957<br>section 2.2 | 1-17 |

☑ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **19 March 2025** | **27 March 2025** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**EP 4 772 661 A1**

<table>
<tr><td colspan="3" align="center">INTERNATIONAL SEARCH REPORT</td><td>International application No.<br><br>**PCT/CN2024/137960**</td></tr>
<tr><td colspan="4">C.    DOCUMENTS CONSIDERED TO BE RELEVANT</td></tr>
<tr><td>Category*</td><td colspan="2">Citation of document, with indication, where appropriate, of the relevant passages</td><td>Relevant to claim No.</td></tr>
<tr><td align="center">A</td><td colspan="2">CN 108823469 A (CIXI INSTITUTE OF BIOMEDICAL ENGINEERING, NINGBO INSTITUTE OF INDUSTRIAL TECHNOLOGY, CHINESE ACADEMY OF SCIENCES et al.) 16 November 2018 (2018-11-16)<br>    description, paragraphs 6 and 10-14</td><td align="center">1-17</td></tr>
<tr><td align="center">A</td><td colspan="2">CN 109097629 A (UNIVERSITY OF SCIENCE AND TECHNOLOGY BEIJING) 28 December 2018 (2018-12-28)<br>    entire document</td><td align="center">1-17</td></tr>
<tr><td align="center">A</td><td colspan="2">CN 108220683 A (UNIVERSITY OF SCIENCE AND TECHNOLOGY BEIJING) 29 June 2018 (2018-06-29)<br>    entire document</td><td align="center">1-17</td></tr>
<tr><td align="center">A</td><td colspan="2">AU 2020104227 A4 (UNIVERSITY OF SCIENCE AND TECHNOLOGY BEIJING) 11 March 2021 (2021-03-11)<br>    entire document</td><td align="center">1-17</td></tr>
</table>

Form PCT/ISA/210 (second sheet) (July 2022)

**INTERNATIONAL SEARCH REPORT**
**Information on patent family members**

International application No.

**PCT/CN2024/137960**

| Patent document cited in search report | | | Publication date (day/month/year) | Patent family member(s) | Publication date (day/month/year) |
|---|---|---|---|---|---|
| CN | 118389906 | A | 26 July 2024 | None | |
| CN | 108823469 | A | 16 November 2018 | None | |
| CN | 109097629 | A | 28 December 2018 | None | |
| CN | 108220683 | A | 29 June 2018 | None | |
| AU | 2020104227 | A4 | 11 March 2021 | None | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **X TONG et al.** *Acta Biomaterialia*, 2018, vol. 82, 197-204
- **E MOSTAED et al.** *Acta Biomaterialia*, 2020, vol. 105, 319-335
- **Z B TANG et al.** *Materials and Design*, 2017, vol. 117, 84-94
- **P K. BOWEN et al.** *Journal of Biomedical Materials Research*, 2018, vol. 106, 245-258